(19) **Europäisches Patentamt**
**European Patent Office**
**Office européen des brevets**

(11) **EP 2 049 012 B1**

(12) **EUROPEAN PATENT SPECIFICATION**

(45) Date of publication and mention
of the grant of the patent:
**15.04.2015 Bulletin 2015/16**

(51) Int Cl.:
*A61B 5/021* *(2006.01)*  *A61B 5/053* *(2006.01)*

(21) Application number: **07805114.1**

(86) International application number:
**PCT/IB2007/052763**

(22) Date of filing: **11.07.2007**

(87) International publication number:
**WO 2008/015598 (07.02.2008 Gazette 2008/06)**

(54) **SENSOR FOR DETECTING THE PASSING OF A PULSE WAVE FROM A SUBJECT´S ARTERIAL SYSTEM**

SENSOR ZUR ERKENNUNG EINER VOM ARTERIELLEN SYSTEM EINER PERSON AUSGEHENDEN IMPULSWELLE

DÉTECTEUR DU PASSAGE D'UNE ONDE D'IMPULSION PROVENANT DU SYSTÈME ARTÉRIEL D'UN SUJET

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **02.08.2006 EP 06118316**

(43) Date of publication of application:
**22.04.2009 Bulletin 2009/17**

(73) Proprietors:
• **Philips Intellectual Property & Standards GmbH 20099 Hamburg (DE)**
Designated Contracting States:
**DE**
• **Koninklijke Philips N.V. 5656 AE Eindhoven (NL)**
Designated Contracting States:
**AT BE BG CH CY CZ DK EE ES FI FR GB GR HU IE IS IT LI LT LU LV MC MT NL PL PT RO SE SI SK TR**

(72) Inventors:
• **MÜHLSTEFF, Jens 5656 AA Eindhoven (NL)**

• **IGNEY, Claudia Hannelore 5656 AA Eindhoven (NL)**
• **THIJS, Jeroen Adrianus Johannes 5656 AA Eindhoven (NL)**
• **PINTER, Robert 5656 AA Eindhoven (NL)**

(74) Representative: **Verweij, Petronella Danielle et al Philips Intellectual Property & Standards P.O. Box 220 5600 AE Eindhoven (NL)**

(56) References cited:
EP-A- 1 350 460  US-A- 3 980 076
US-A- 4 452 252  US-A- 5 309 916
US-A1- 2002 035 337

• 'ORTHOGONAL-COILS RECEIVER: A CONFIGURATION FOR IMPROVING THE POSITION TOLERANCE OF COUPLED MORPHOGNOSTIC COILS' MEDICAL AND BIOLOGICAL ENGINEERING AND COMPUTING vol. 21, no. 2, 01 March 1983, SPRINGER, HEILDELBERG, DE, pages 224 - 226, XP009008216 ISSN: 0140-0118

**Description**

The present invention relates to a sensor and a method for detecting the passing of a pulse wave from a subject's arterial system. Furthermore the invention relates to a non-invasive measuring system, being adapted to be attached to the exterior of the subject's body, and to methods for determining various vital signs of a subject.

Blood pressure (BP) is one of the most important physiological parameters and plays a major role in medical diagnostics, prevention as well as in disease management systems. It is an independent risk factor for cardiovascular disease and renal disease. In 2006 there were 65 million adults in the US having hypertension with systolic pressure >140 mmHg and diastolic pressure >90 mmHg and/or use antihypertensive drugs. Additionally one-quarter of US-adults have "prehypertension". These numbers indicate that hypertension causes a strong social burden and new strategies in blood pressure monitoring and therapy have been proposed. Besides spot measurements at hospitals it is now recommended to extend blood pressure measurements to a home based continuous monitoring.

There are several established methods and devices providing BP values measured non-invasively: e.g. using sphygmomanometers (auscultatory method), using oscillometric techniques, that is the most wide-spread technique for self measurement, tonometry or the finger cuff method of Penaz. All approaches use a cuff and an external pressure must be applied to the subject's body.

Unsupervised BP measurements are prone to measurement artifacts due to ill-defined measurement conditions (like varying room temperature, cuff position and cuff size) and/or patient non-compliance (no physical activity 5 min before the measurement, wrong position of the patient).

Recent research has shown a good correlation between the arterial BP and the velocity of pulse waves (PWV) propagating in the arterial tree, which allows a beat-to-beat determination of BP. This technique doesn't require a cuff for measurements and no external pressure to the subject's body is required. A simplistic relation of BP and PWV in arteries can be derived from the Moens-Korteweg-relation, which is known from hydrodynamic theory:

$$c = \sqrt{\frac{hE_t}{2\rho R}} \qquad \text{Eq. 1}$$

in which c denotes the pulse wave velocity, $E_t$ denotes the tangential elasticity module of the artery, $\rho$ denotes the blood density, R denotes the radius of artery and h denotes the artery wall thickness. The relation of BP and PWV is given via the dependency of the elasticity modulus $E_t$ from the BP, which has been described e.g. in US 4,425,920.

The PWV can be determined by measuring the time of a pressure pulse traveling a certain distance in the arterial system. This propagation time is called pulse transit time (PTT) and from the prior art there are a number of methodologies known how the PTT can be measured: e.g. by measuring the time-difference of a pulse passing two points at a distance d or by measuring the time-difference between the R-peak in an electrocardiography (ECG)-signal and a passing pulse in an artery at a certain body position from a plethysmography-sensor. PTT can then be used as a surrogate for PWV.

From the prior art, a large number of PTT measurement set-ups are known, e.g.

-    the combined use of ECG and photoplethysmography (PPG), wherein the PTT is given by time-difference between R-peak and characteristic points in PPG,
-    the combined use of ECG and Laser-Doppler-Flow measurement,
-    the combined use of ECG and bio-impedance measurement at one arm (IPG, impedance plethysmography), wherein the PTT is given by time-difference between R-peak and characteristic points in IPG (see e.g. US patent 6,648,828)"
-    the combined use of ECG and ultrasound flow measurement,
-    the combined use of impedance cardiography (ICG) of the thorax and IPG, or
-    the measurement of "local" PTT values between two points at a distance d with a first PPG measured e.g. at the wrist and a second PPG e.g. at the finger.

All these methods have several disadvantages. Ultrasound-sensors need contact gel for proper function. Impedance and ECG measurements have to be done with electrodes, which have normally to be glued to the skin. PPG and Laser-Doppler sensors have to be placed at body points under which arteries are close to the skin. The measurements of "local" PTT values have very little accuracy due to the small distance of wrist to finger, which is caused by the requirement of arteries close to the skin.

PTT measurements based on ECG-signals have the disadvantage, that the electrical function of the heart is related to a mechanical measure. The pre-ejection period (PEP), the time of iso-volumetric contraction of the heart muscle, can have strong influence on PTT without a relation to the BP.

US5,309,916 representing the closest prior art and disclosing the preamble of claim 1 describes a device for measuring blood pressure that includes a sensor arrangement which is releasably attached to the exterior of a body and which is electrically conductively connected with electronic circuit. The sensor arrangement and the circuit are configured to determine, in at least one measuring region of the body, a valve which is a measure for a variable that changes periodically over time in the rhythm of the pulse beat and which is correlated with the blood pressure. This variable may, for example, be the flow velocity and/or flow quantity and/or the volume of the arterial blood and/or a cross-sectional dimension and/or the flow cross section area of an arterial blood vessel. The sensor and circuit further determine a value which is a measure for the pulse wave velocity.

EP1350460 describes a vital sign detection sensor and sensor controlling device in which a sensor device is comprised of a power generating unit that takes in energy externally (a power carrier radio wave) and generates direct current power from such energy, a vital sign detecting unit that detects a vital sign of a user while being supplied with the direct current power from the power generating unit, and a vital sign transmitting unit that transmits the detected vital sign cordlessly while being supplied with the direct current power from the power generating unit.

It is an object of the present invention to provide an easy-to-use technique for measuring BP and/or other vital signs of a subject, in which the above-mentioned disadvantages are avoided.

This object is achieved according claim 1 by a sensor for detecting the passing of a pulse wave from a subject's arterial system, the sensor being adapted to be located at a sensing position on the exterior of the subject's body, characterized in that the sensor comprises a number of electrical coils for generating an inductive coupling to the subject's body in a way that the properties of said inductive coupling change if a pulse passes a screened volume underneath the sensing position, and detecting said property changes of the inductive coupling.

This object is also achieved according to the present invention by various non-invasive measuring systems, which uses such a sensor, as described below in more detail.

Furthermore this object is also achieved according to the present invention by a computer program to be executed in a computer, which analyses the signals from the sensor for detecting the passing of a pulse wave from a subject's arterial system, during which an inductive coupling between a number of electrical coils and the subject's body is generated in a way that the properties of said inductive coupling change if a pulse passes a screened volume underneath the sensing position, the program comprising computer instructions to detect said property changes of the inductive coupling, when the computer program is executed in a computer. The technical effects necessary according to the invention can thus be realized on the basis of the instructions of the computer program in accordance with the invention. Such a computer program can be stored on a carrier such as a CD-ROM or it can be available over the internet or another computer network. Prior to executing the computer program is loaded into the computer by reading the computer program from the carrier, for example by means of a CD-ROM player, or from the internet, and storing it in the memory of the computer. The computer includes inter alia a central processor unit (CPU), a bus system, memory means, e.g. RAM or ROM etc., storage means, e.g. floppy disk or hard disk units etc. and input/output units. Alternatively, the inventive method could be implemented in hardware, e. g. using one or more integrated circuits.

A basic idea of the present invention is to use the principles of magnetic induction in order to detect the passing of a pulse wave. The proposed sensor placed on a certain body-part detects the change of certain parameters, which represents the passing of a pulse. These parameters are blood volume, geometry and conductivity. Since the conductivity of blood depends on the blood velocity, the conductivity of blood changes, if a pulse wave passes. At the same time, the geometry of the blood vessel changes because of the passing of the pulse wave (enlargement and contraction) and thus the blood volume within the screened volume changes. In other words, changes of the blood volume within the screened volume as well as geometrical changes and conductivity changes underneath the sensing position, i.e. underneath the position of the sensor, within the screened body volume, are sensed. For sensing these changes, the sensor comprises a number of electrical coils, i.e. one or more electrical coils, together with an appropriate electronic driving circuit. Pulse waves are detected using the principles of magnetic induction. The above mentioned changes result in a cumulated change of the magnetic coupling between the subject's body and the sensor coil(s), which are used for detecting the pulse. Based on the detected pulse waves, PTT and/or PWV values can be determined. These values can be used for determining BP of the subject, the pulse wave of which has been detected.

With the present invention a contactless, non-invasive measurement of BP and other vital parameters is possible. No cuff is needed. The proposed sensor does not have to be glued to the skin and needs no contact gel. The positions of the sensors are not restricted to the position of arteries close to the skin. Pulse waves from arteries deeper in the body can be detected as well.

Additionally, if the sensor is placed around the heart-position, the signal of the sensor contains information on the instantaneous mechanical movement of the heart during a pumping cycle. This enables an accurate measurement of the point in time, in which a pulse wave starts propagating from the heart to the outside arteries. Therefore if this sensor is used as proximal sensor for BP measurements the inclusion of the PEP is avoided.

The present invention can be used e.g. for non-invasive measurement of pulse rate, respiration rate, pulse transit time as well as for non-invasive and continuous determination of arterial blood pressure.

Since the suggested sensor can be used for moveable and wearable measuring systems, an easy-to-use BP measuring procedure can be implemented. The present invention can be used for unsupervised, long-term continuous monitoring of BP and other vital signals, like heart rate and respiration rate.

These and other aspects of the invention will be described in detail hereinafter, by way of example, with reference to the following embodiments and the accompanying drawings; in which:

Fig. 1     shows a general principle of measurement,
Fig. 2     shows an equivalent circuit,
Fig. 3     shows an experimental setup with two coils,
Fig. 4     shows a relative signal amplitude of a receiving coil depending on a radius change of an artery,
Fig. 5     shows a relative voltage-change in receiving coils when a blood volume pulse passes the coil arrangement,
Fig. 6     shows an experimental setup with three coils,
Fig. 7     shows a first circuitry of a single coil arrangement,
Fig. 8     shows a second circuitry of a single coil arrangement,
Fig. 9     shows a current-frequency dependency in a single coil embodiment,
Fig. 10    shows a current-frequency dependency in a single coil embodiment,
Fig. 11    shows a third circuitry of a single coil arrangement,
Fig. 12    shows the switching between "sample" mode and "hold" mode in circuitry 102,
Fig. 13    shows a circuitry of a dual coil arrangement,
Fig. 14    shows an example of a measuring device, and
Fig. 15    shows another example of a measuring device.

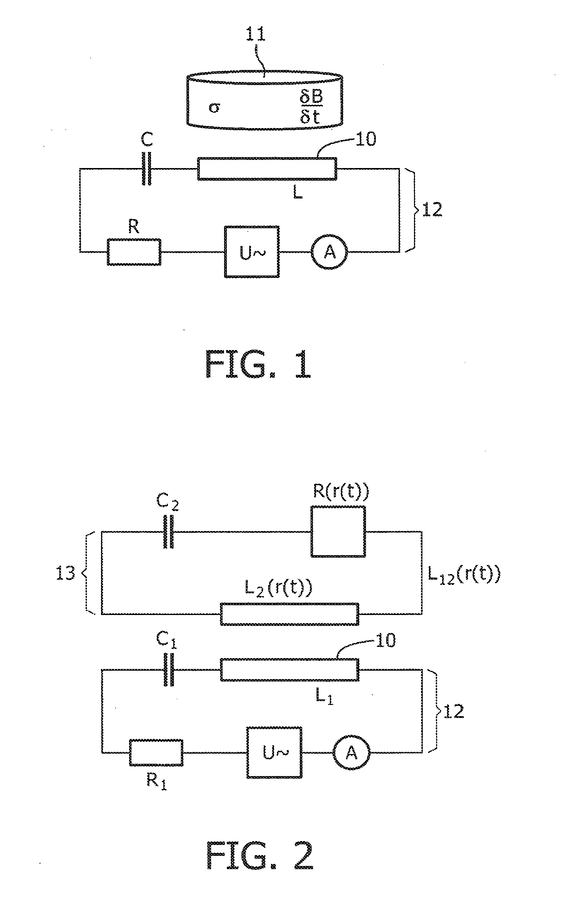

The proposed invention is based on inductive methods. The general principle is shown in Fig. 1 for a single coil embodiment. A magnetic field produced by the current within a measuring coil 10 induces eddy currents in the conductive tissue 11 of the subject's body to be screened (induction of eddy currents in a volume conductor).

The equivalent circuit for modeling a measurement system with single coil setup as shown in Fig. 2 describes the situation according to Fig. 1 using standard electrical elements. The measuring coil 10 of the primary circuit 12 is coupled by induction coefficients $L_{1i}$ to the body circuit 13, which are primarily defined by the electrical properties of the tissue, vessels and bones inside the screened volume 11 of the subject's body. The resonance frequency and impedance of the electrical circuits 12, 13 varies due to changes within the screened body volume 11. Blood for instance shows different resistances at different flow velocities during a heartbeat due to the alignment of erythrocytes. Additionally there are geometrical changes, because vessels inflate or deflate. These changes are detected and used for determining the passing of a pulse wave in the screened volume. The following equations can be used for modeling the measurement system:

$$L_1 \ddot{I}_1 + L_{12} \ddot{I}_2 + R_1 \dot{I}_1 + \frac{I_1}{C_1} = \dot{U} \qquad \text{Eq. 2}$$

$$L_2 \ddot{I}_2 + L_{12} \ddot{I}_1 + R_2 \dot{I}_2 + \frac{I_2}{C_2} = 0 \qquad \text{Eq. 3}$$

with $L_{12}$ given by the following equation for i=1, j=2:

$$L_{ij} = \frac{\mu_0}{4\pi I_i I_j} \iint_{V,V'} \frac{\vec{j}_i \cdot \vec{j}'_j}{|\vec{r} - \vec{r}'|} d\tau d\tau' \qquad \text{Eq. 4}$$

Mathematically the current amplitude in primary circuit 12 according to the equivalent circuit in the single coil arrangement according to Fig. 2 can be expressed for a simplified cylindrical problem according to the following expression:

$$\hat{I} = \cfrac{i\omega U_0}{\cfrac{1}{C_1} + \omega^2 (L_{11} + \cfrac{1}{2\pi} \sum_{i=1}^{k} \cfrac{\sigma_i(t) L_{1i}^2(t)}{r_i}) + i\omega R_1} \qquad \text{Eq. 5}$$

in which $U_0$ denotes the amplitude of the driving oscillator, $R_1$ denotes the resistance of the primary circuit, $C_1$ denotes the capacitance in the primary circuit, $L_{11}$ denotes the self inductance of the primary coil, $L_{li}$ denotes the coupling inductance of the primary coil and circle eddy currents, $\sigma$ denotes the conductivity in the secondary circuit (describing tissue conductivities), and $\omega$ denotes the angular frequency. As it can be seen according to Eq. 5 the measurable current $\hat{I}$ depends on the coupling coefficients $L_{li}(t)$ and the conductivity changes $\sigma(t)$.

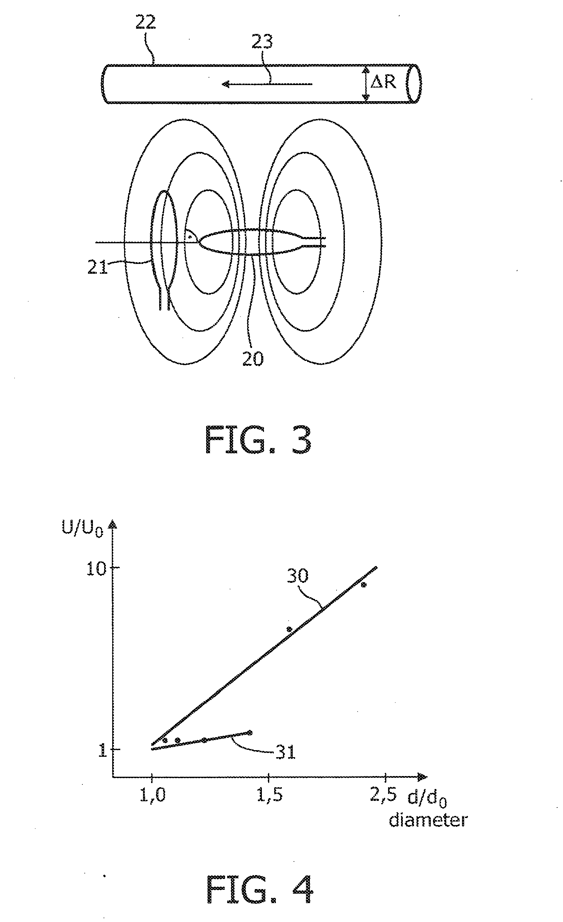

Experimental and numerical methods have been used for modeling a specific sensor configuration for use at a subject's wrist. An experimental setup evaluating sensitivity of radius changes of an artery is schematically shown in Fig. 3. Two coils 20, 21, the axes of which are perpendicular to each other, form a coil arrangement. The field coil 20 $L_e$ produces a primary magnetic field, which is screened by the sensing or receiving coil 21 $L_m$, which is located perpendicular to $L_e$. An artery 22 has been modeled by an elastic tube filled with water having conductivity similar to that of blood. The direction of blood flow is illustrated by arrow 23. The tube radius R was changed via a pressure increase in the tube. The following setup parameter has been used: $\omega = 2\pi \cdot 4$ MHz, radius of sensing coil $L_m = 5$ cm, radius of primary coil $L_e = 5$ cm. The induced voltage in the receiving coil $L_m$ was measured via the well-known lock-in method.

In Fig. 4 experimental results for the setup shown in Fig. 3 are illustrated. In particular, Fig. 4 shows the measured relative signal amplitude (relative voltage change $U_m/U_{m0}$ ($U_{m0}$ for $R=R_0$)) of the receiving coil $L_m$ depending on the relative change of tube radius with respect to two different background conductivities. As a first background (first curve 30) air is used (conductivity 0 $Sm^{-1}$). As a second background (second curve 31) conductive water is used, simulating fat conductivity 0.04 $Sm^{-1}$. It can be seen that even in a background of fat a measurable effect can be observed due to a radius change of the tube 22. The dimension of the setup can be scaled down for different body locations and realistic artery geometries e.g. femoralis or carotis.

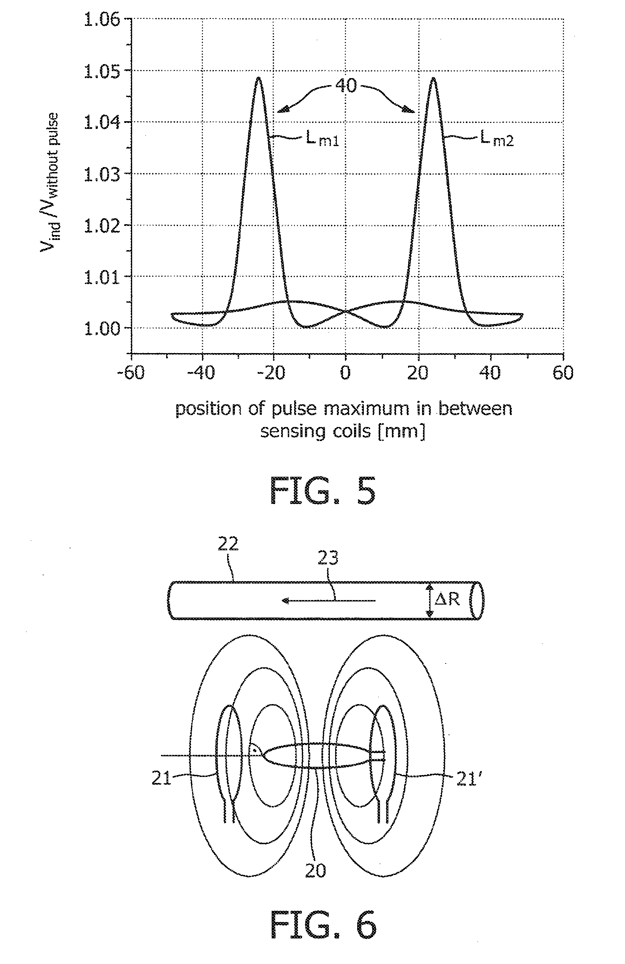

In Fig. 5 results of a numerical simulation are illustrated. The simulation has been carried out using a model having the dimension of a realistic embodiment. The setup for this simulation was similar to Fig. 3 with an additional receiving coil $L_{m2}$ 21' opposite to the first receiving coil $L_{m1}$ 21. This setup is shown schematically in Fig. 6. The following parameter has been used: radius of primary coil $L_e = 15$ mm, radius of sensing coils $L_{mi} = 2.5$ mm (normal aligned), distance of $L_e$ to fat= 5 mm, distance of $L_{m1}$ to $L_{m2} = 50$ mm, artery radius= 1.5 mm, pulse cube radius= 2.5 mm, distance air/artery= 1.5 mm, background fat= 0.04 $Sm^{-1}$, blood conductivity= 0.7 $Sm^{-1}$.

Fig. 5 shows the calculated relative voltage-change in both receiving coils $L_{m1}$, $L_{m2}$ when a blood volume pulse passes the arrangement for a background of fat. There is a maximum relative voltage change of about 5 % during the passage of the blood volume pulse. Due to the symmetrical arrangement there are two similar voltage differences 40 in both coils. As it can be seen in Fig. 5 this voltage change can easily be detected. Thus, the proposed method can be used to detect blood flow pulses in a very comfortable way.

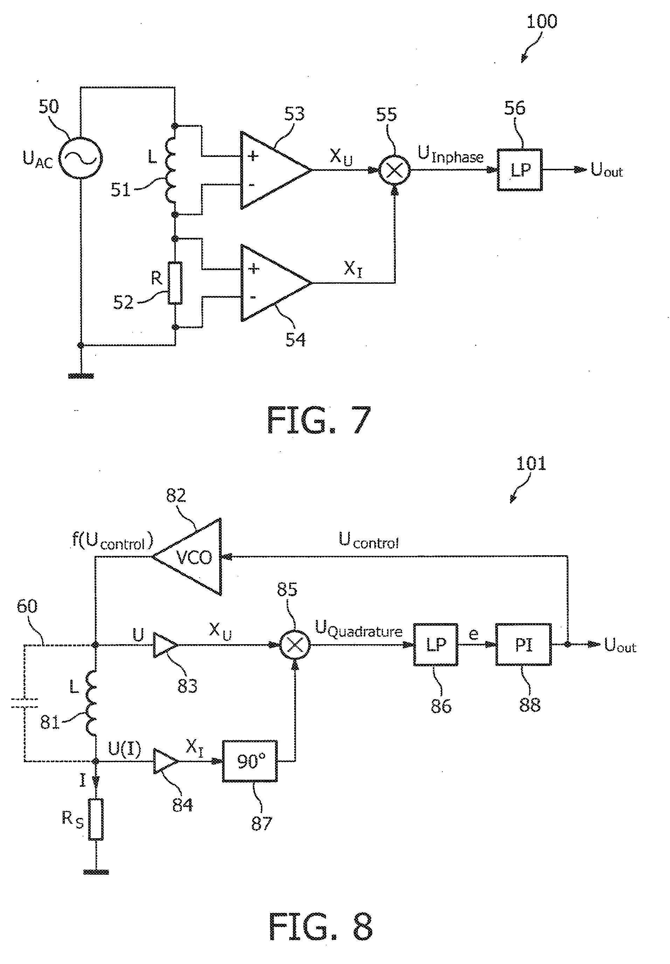

For implementing the principle of magnetic induction a single coil arrangement or a dual coil arrangement can be used. If a single coil setup is used, the measuring is based on changes of the coils parameter due to the influence of the screened body volume. In particular a change of the phase angle between the coil voltage and the current through the coil can be observed in case an electric conducting material (like blood in the form of a pulse wave) passes the coil's position. Fig. 7 illustrates a control circuit 100 for a single coil setup for measuring the phase angle. An AC supply point 50 impresses a voltage into a serial connection of a measuring coil L 51 and a resistor R 52. The voltage drop on the resistor R 52 is directly proportional to the current through the coil L 51. Using a first differential amplifier 53 the voltage drop on the coil L 51 is determined. Using a second differential amplifier 54 the voltage drop on the resistor R 52 is determined, which is a measure for the current through the coil L 51. The output signals of the differential amplifiers 53, 54 are given by

$$x_U(t) = A_U \cdot \sin(\omega t) \qquad \text{Eq. 6}$$

$$x_I(t) = A_I \cdot \sin(\omega t + \varphi) \qquad \text{Eq. 7}$$

In the above equations $\omega$ denotes the angular frequency of the feeding alternating current $U_{AC}$, $\varphi$ denotes the phase angle between voltage and current (to be determined), and $A_U$ and $A_I$ are the amplification factors of the differential amplifiers 53, 54. A mixer 55 is used to generate the product of voltage $X_U$ (which is proportional to the voltage U) and voltage $X_I$ (which is proportional to the current I). This product is denoted $U_{inphase}$.

$$U_{inphase} = A_U \sin(\omega t) A_I \sin(\omega t + \varphi) \qquad \text{Eq. 8}$$

$$U_{inphase} = \frac{A_U A_I}{2} \left[ \cos(\varphi) - \cos(2\omega t + \varphi) \right] \qquad \text{Eq. 9}$$

Using a low-pass filter LP 56 the higher frequency ($2\omega$ in Eq. 9) is eliminated and a resulting output signal $U_{out}$ is generated.

$$U_{out} = \frac{A_U A_I}{2} \cos\varphi \qquad \text{Eq. 10}$$

In case of an "ideal" inductance the coil voltage leads the coil current by $\phi$=90°. In this "ideal" case the output signal $U_{out}$ is zero because of cos(90°)=0. Because of the inductive coupling of coil L 51 and electrical conducting tissue (not shown in Fig. 7), the phase angle $\phi$ is decreased and the output signal $U_{out}$ is not zero. In other words, the blood pulse within an artery, representing a blood volume with varying throughput, passing the coil L 51, modulates the amplitude of the output signal $U_{out}$.

In practical operation no pure inductive measuring coil L 51 is obtained. Because of the coil supply lines 60, there are parasitic couplings, see Fig. 8. These couplings are small, however, resulting in a capacitory effect. The combination of measuring coil L 81 and parallel connected parasitic capacitor form a resonant circuit. Typically, the self-resonant frequency of such a measuring coil L 81 is some MHz. In other words, the self-resonant angular frequency is in the frequency range of the measuring angular frequency $\omega$.

The electromagnetic coupling to the passing pulse wave attenuates the resonance amplitude and detunes the resonance frequency of the measuring setup. These effects can be used for pulse detection as well.

A way of operating a single coil setup at the self-resonant frequency of the coil is described below. For this purpose a closed loop control circuit 101 is used. Instead of an AC supply point $U_{AC}$ a voltage-controlled oscillator 82 with variable frequency is used. The oscillator 82 feeds a parallel resonant circuit, which is formed by the measuring coil L 81 and the parasitic capacity (supply lines 60). In Fig. 8 basic buffer amplifier 83, 84 are used instead of the differential amplifiers, resulting in low wiring requirements. However, differential amplifiers can be used as well.

In the closed loop control circuit illustrated in Fig. 8 an "error" voltage e is provided as resulting signal of mixer 85 and low-pass filter 86. The aim is to adjust this error value e to zero. In contrast to the embodiment illustrated in Fig. 7, in which the resulting voltage of mixer 55 and low-pass filter 56 changes to zero if coil voltage and coil current show a phase angel of 90°, in the embodiment illustrated in Fig. 8 an additional 90° phase shifter 87 is employed in a way that voltage e equals zero if voltage and current of the coil L 81 are in phase (phase angle=0°). In an oscillating circuit this is the case if the operating frequency equals the resonance frequency. As long as the error voltage e is not zero, the PI controller 88 receiving the error voltage e regulates its output voltage $U_{out}$ such that its input voltage becomes zero (e=0). In other words, the PI controller 88 uses the error voltage e to generate a control voltage $U_{control}$ for the voltage controlled oscillator 82. The oscillator 82 is controlled until the error voltage e equals zero, i.e. the present resonance frequency is reached.

Instead of a PI controller 88 (proportional integral controller) a P controller (proportional controller) can be used. In this case the error voltage e can be adjusted to a minimum only, the value of which depends on the amplification factor of the P controller. A PI controller with integral part however integrates lowest error voltages e. Other controllers known in the state of the art can also be used.

An output value of the illustrated setup is the control voltage $U_{control}$ of the oscillator 82. If the resonance frequency changes because of a pulse wave passing the coil L 81, the control loop control circuit tunes the oscillator 82 accordingly, and the control voltage $U_{control}$ of the oscillator 82 changes.

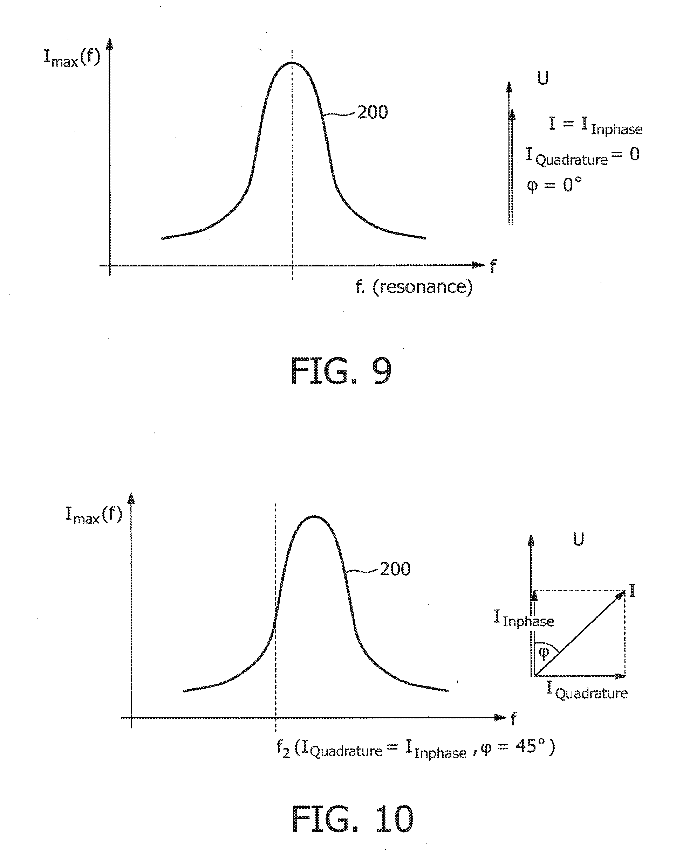

Fig. 9 illustrates a typical current-frequency dependency in a single coil embodiment (parallel oscillating circuit) in case of $\varphi$= 0° (resonance frequency $f_I$). In case of resonance the phase angle is zero, i.e. voltage and current are in phase. In other words, current I consists of the part $I_{inphase}$ only. As a second output value in case of resonance the signal $X_I$ can be used, as illustrated in Fig. 8. Signal $X_I$ corresponds to the amplitude of the resonance curve, representing the attenuation of the oscillating circuit. If the attenuation of the oscillating circuit is high, e.g. due to electrical conductive material, like blood, the resonance curve shows a low amplitude.

The setup as illustrated in Fig. 8 allows the determination of the present resonance frequency and the determination of the attenuation of the oscillating circuit, both values depending on the presence of electrical conducting material (e.g. a passing pulse wave) close to the measuring coil L 81.

In another embodiment a measurement setup is used, which in particular is of advantage in case of very high measuring sensitivity. Again the setup is built as a closed loop control circuit 102, in which the oscillator 92 is controlled such, that instead of a resonance a certain point on the edge of the resonance curve 200 is reached. This certain point is defined such that the part $I_{inphase}$ of current in phase with the coil voltage is equal in size to the part $I_{quadrature}$ of current, which shows a phase angel of 90° to the coil voltage, as illustrated in Fig. 10. A typical current-frequency dependency in a single coil embodiment (parallel oscillating circuit) in case of $\varphi=45°$ (frequency $f_2$) is shown.

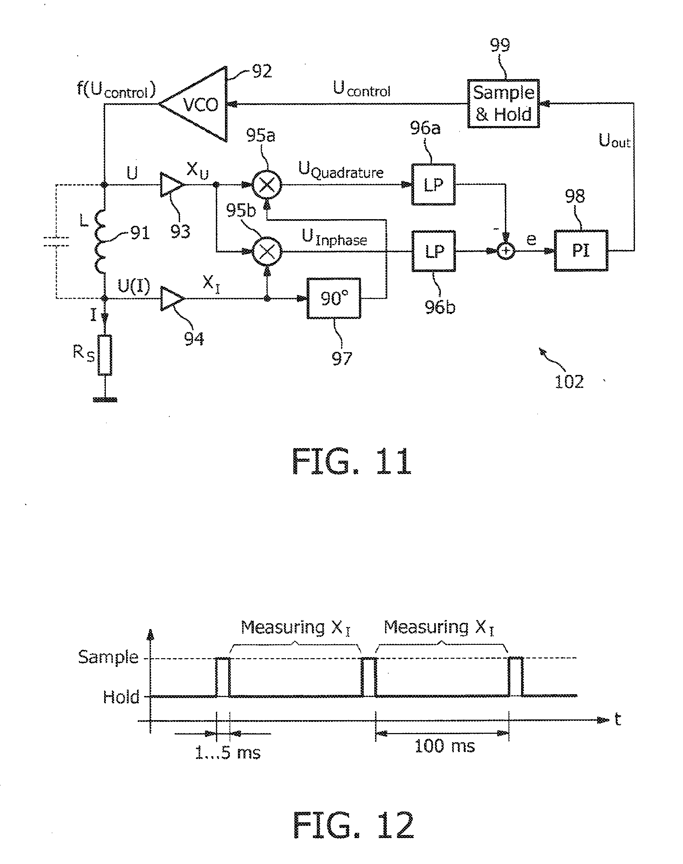

The closed loop control circuit 102 used in this embodiment is adapted in a way that the difference between $I_{inphase}$ and $I_{quadrature}$ is used as the error value to be minimized. If both parts of current show the same size, the difference equals zero. In Fig. 11 a setup is shown for operating on the edge of the resonance curve 200. In this embodiment the setups as shown in Fig. 7 and 8 are combined. A first mixer 95a determines the components of the coil current, which are in phase with the coil voltage, as known from Fig. 7. A second mixer 95b determines the component of the coil current, which show a 90° phase shift to the coil voltage, as known from Fig. 8. Both components are subtracted from each other and the resulting value is fed to the PI controller 98 as an error value. The PI controller 98 generates the control voltage for the voltage controlled oscillator. The PI controller 98 regulates its output voltage $U_{out}$ such that its input voltage becomes zero (e=0).

In contrast to the embodiments described above, additionally a sample & hold element 99 is employed. The sample & hold element 99 is located between the PI controller 98 and the voltage controlled oscillator 92 and serves as a closed switch, if the sample & hold element 99 operates in "sample" mode. In other words, the output voltage $U_{control}$ of the PI controller 98 is given to the control input of the oscillator 92. If the sample & hold element 99 operates in "hold" mode, the sample & hold element 99 interrupts the direct connection between the PI controller 98 and the oscillator 92. At the same time the sample & hold element 99 provides ("holds") on its output the last valid voltage value.

In other words, in the "sample" mode the closed loop of the control circuit 102 will be closed and the inflection point on the edge of the frequency curve 200 will be used as operating frequency, and in the "hold" mode the closed loop of the control circuit 102 will be opened in a way that the oscillator 92 oscillates with the last setup frequency.

In order to carry out a measurement the oscillator 92 is set up on the inflection point of the edge of the present frequency curve 200 using the "sample" mode. In a next step the sample & hold element 99 is switched to the "hold" mode. Because the inflection point is the steepest point on the edge of the resonance curve 200, a small shift of the coil's natural resonance, e.g. due to a passing pulse wave, results in a large effect on the amplitude of the coil current to be measured. In order to detect a passing pulse wave, the voltage $X_I$ is measured, which represents the coil current.

In practice there is a periodical switching between the "sample" mode and the "hold" mode, in order to provide a quasi-continuous measurement, during which the readjusting during the "sample" mode is done within some milliseconds. An example of such a switching between "sample" mode and "hold" mode illustrates Fig. 12.

In "sample" mode slow changes of the environment, e.g. a changing coupling of the measuring coil to the part of the subject's body which is used for pulse detection, would be compensated, whereas fast measuring effects, e.g. caused by a passing pulse wave, would be acquired in a quasi-continuous way during the "hold" mode.

In order to control the different modes of operation, a preferred control strategy is to provide a short switch from the "hold" mode to the "sample" mode only in case of a significant deviation of the coupling behaviour due to a changing measuring environment. Such a deviation is determined by observing the output of the PI controller 98. In "hold" mode the PI controller 98 verifies the error value, i.e. the difference between $I_{inphase}$ and $I_{quadrature}$. As long as this difference is below a certain threshold, the measurement is "on edge". The threshold has to be set high enough, such that the short peaks of the error value caused by passing pulse waves do not lead to a switch to the "sample" mode.

In a single coil setup, as described above, the electrical coil parameters change due to neighbouring electrical conductive material, e.g. a pulse wave. A change of coil parameters is detected by measuring of coil voltage and coil current and by determining the phase difference.

A measurement system with dual coil setup is illustrated in Fig. 3. In this setup the coupling between two separate coils 20, 21 is changed due to neighbouring electrical conductive material, e.g. a pulse wave. Without electrical conductive material being in the neighbourhood of the coils 20, 21 the net flux through the receiving coil $L_m$ 21 is zero due to the symmetry of the coil arrangement, i.e. no voltage is induced in the receiving coil $L_m$ 21. If an electrically conductive material is in the neighbourhood of the coils the magnetic field of the field coil $L_e$ 20 is distorted and the net flux through the receiving coil $L_m$ 21 does not equal zero. In other words, a voltage is induced.

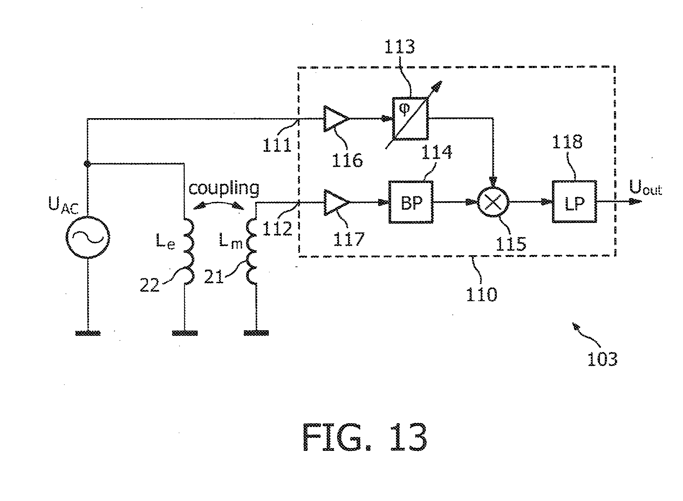

In contrast to the single coil setup, the aim of the measuring setup is not to examine the phase difference of two signals. Instead, a very small signal in a very "noisy" environment has to be measured. A known method for such a measurement is the so called lock-in method. In Fig. 13 a setup for a control circuit 103 is shown, in which a lock-in amplifier 110 is used to evaluate the signals of the dual coil setup.

The lock-in amplifier 110 comprises two signal input channels. The first input channel (reference input) 111 is used for a reference signal and the second input channel (measuring input) 112 is used for a measuring signal. As reference signal the alternating voltage $U_{AC}$ of the field coil $L_e$ 20 is used. The measuring signal is the voltage which is induced in the receiving coil $L_m$ 21.

Without any coupling between the two coils 20, 21, the induced voltage is zero. If an electrically conductive material is in the neighbourhood of the coils 20, 21, a very small alternating voltage is induced in the receiving coil $L_m$ 21. This alternating voltage exhibits the same frequency as $U_{AC}$. Amplitude and phase of the voltage depend on the coupling between the two coils 20, 21.

In order to compensate the phase shift between reference signal and measuring signal, the lock-in amplifier 110 comprises an adjustable phase shifter 113, which is adapted in a way that to the mixer 115 both signals are provided with the same phasing. In this way, a maximal output voltage $U_{out}$ can be obtained after the low-pass filter 118, which is provided after the mixer 115.

$$U_{out\_max} = \frac{A_{measurement} \cdot A_{reference}}{2} \qquad \text{Eq. 11}$$

Eq. 14 is equivalent to Eq. 13, which is maximal for $\varphi=0°$. An advantage of the lock-in technique is that interfering frequencies and noise exhibiting undefined or changing phasing with regard to the reference signal averages to zero after the mixer 115. In the amplifier 110 the reference signal passes a buffer 116 in order to reach the phase shifter 113 and the measuring signal passes a buffer 117 and a band-pass filter 114 in order to reach the mixer 115.

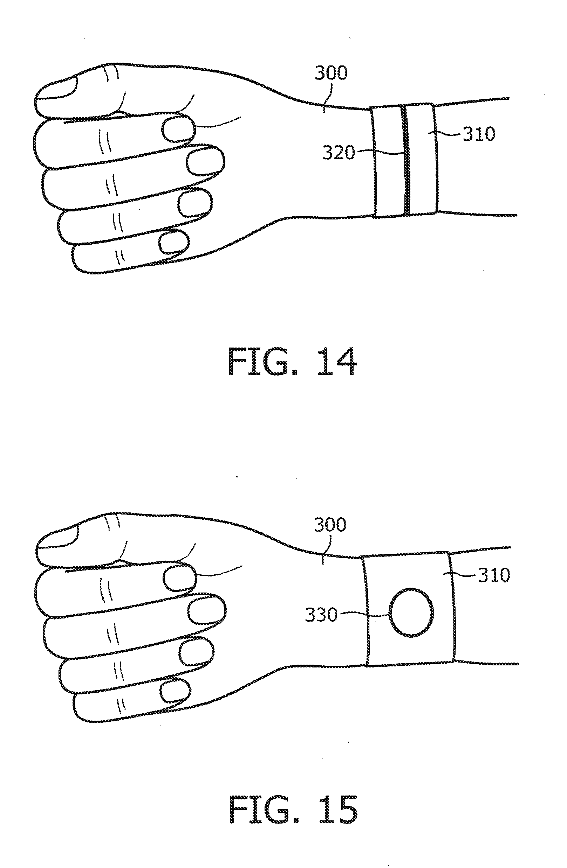

Figs. 14 and 15 illustrate two different embodiments of a non-invasive mobile measuring system comprising a sensor as described above. The measuring system comprises a wristband 310 or bracelet or the like, into which the sensor coil(s) 320, 330 are integrated together with the circuitry and a power supply, e.g. a small battery. In addition a display (not shown) can be provided for displaying heart rate or other physiological parameters to the user. In a first embodiment a larger single coil is arranged in a way that it embraces the user's wrist 300 (Fig. 14). In a second embodiment a small single coil 320 is arranged on the exterior of the subject's body, at a certain place of the user's wrist 300, surrounding a spot of some centimeter in diameter (Fig. 15).

Instead of a wristworn device, other devices can be provided to wear the measuring device at different parts of the body, e.g. on the chest, the waist, the ankle etc. Two or more of such devices can be worn simultaneously in order to provide a BP measurement or a multiple parameter measurement. Alternatively, the measuring system according to the present invention can be adapted to be part of a garment or another piece of clothing, e.g. an underwear etc.

The measuring device preferably comprises a built-in analysing unit, comprising a microprocessor or the like in order to execute an analysing software. The analysing software is adapted to determine, based on the detected pulse waves, PTT and/or PWV values. Furthermore the analyzing software is adapted to determine BP values of the subject wearing the measuring device. Depending on the number of sensors used and the sensing positions, different vital parameters can be determined, e.g. heart rate, respiration rate etc.

All appliances described above are adapted to carry out the method according to the present invention. All circuitry 100, 101, 102, 103, in particular all programmable devices, are constructed and programmed in a way that the procedures for obtaining data and for data processing run in accordance with the method of the invention. In particular the controller are adapted for performing all tasks of calculating and computing the measured data as well as determining and assessing results. This is achieved according to the invention by means of a computer software comprising computer instructions adapted for carrying out the steps of the inventive method, when the software is executed in a processing unit, controller and/or circuitry. The processing unit itself may comprise functional modules or units, which are implemented in form of hardware, software or in form of a combination of both.

It will be evident to those skilled in the art that the invention is not limited to the details of the foregoing illustrative embodiments, and that the present invention may be embodied in other specific forms. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein. It will furthermore be evident that the word "comprising" does not exclude other elements or steps, that the words "a" or "an" do not exclude a plurality, and that a single element, such as a computer system or another unit may fulfil the functions of several means recited in the claims. Any reference signs in the claims shall not be construed as limiting the claim concerned.

REFERENCE NUMERALS

10     measuring coil
11     tissue

| | |
|---|---|
| 12 | primary circuit |
| 13 | body circuit |
| 20 | field coil |
| 21 | measuring coil |
| 22 | artery |
| 23 | direction of blood flow |
| 30 | curve |
| 31 | curve |
| 40 | voltage difference |
| 50 | supply point |
| 51 | measuring coil |
| 52 | resistor |
| 53 | differential amplifier |
| 54 | differential amplifier |
| 55 | mixer |
| 56 | low-pass filter |
| 60 | supply line |
| 81 | measuring coil |
| 82 | oscillator |
| 83 | amplifier |
| 84 | amplifier |
| 85 | mixer |
| 86 | low-pass filter |
| 87 | phase shifter |
| 88 | PI controller |
| 92 | oscillator |
| 93 | buffer |
| 94 | buffer |
| 95 | mixer |
| 96 | low-pass filter |
| 97 | phase shifter |
| 98 | controller |
| 99 | sample & hold element |
| 100 | control circuit |
| 101 | control circuit |
| 102 | control circuit |
| 103 | control circuit |
| 110 | lock-in amplifier |
| 111 | input channel/reference channel |
| 112 | input channel/measurement channel |
| 113 | phase shifter |
| 114 | band-pass filter |
| 115 | mixer |
| 116 | buffer |
| 117 | buffer |
| 118 | low-pass filter |
| 200 | resonance curve |
| 300 | wrist |
| 310 | wristband |
| 320 | measuring coil |
| 330 | measuring coil |

## Claims

1. A sensor for detecting the passing of a pulse wave from a subject's arterial system (22), the sensor being adapted to be located at a sensing position on the exterior of the subject's body, wherein the sensor comprises:

- a number of electrical coils (20, 21, 21') for generating an inductive coupling to the subject's body in a way that the properties of said inductive coupling change if a pulse wave passes a screened volume underneath the sensing position, and
- a circuit (100, 101, 102, 103) connected to the number of electrical coils, said circuit being adapted to detect said property changes of the inductive coupling, **characterised in that** the number of electrical coils comprises a field coil (20) for producing a primary magnetic field and at least one receiving coil (21,21') for screening the primary magnetic field, the axis of the at least one receiving coil being perpendicular to the axis of the field coil.

**2.** The sensor as claimed in claim 1, **characterized in that** the at least one receiving coil (21, 21') comprises a single electrical coil, the electrical properties of which being changed, if a pulse wave passes the sensing position.

**3.** The sensor as claimed in claim 1, **characterized in that** the at least one receiving coil (21, 21') comprises two separate electrical coils, the electrical properties of which being changed, if a pulse wave passes the sensing position.

**4.** A non-invasive measuring system, being adapted to be attached to the exterior of the subject's body, **characterized in that** it comprises one sensor as claimed in claim 1, the system being adapted to provide information about the heart rate of the subject.

**5.** A non-invasive measuring system, being adapted to be attached to the exterior of the subject's body, comprising two sensors as claimed in claim 1, the sensing positions of said sensors being spaced apart, the system being adapted to provide information about the blood pressure of the subject.

**6.** Method for detecting the passing of a pulse wave from a subject's arterial system (22), the method comprising the steps of:

- generating an inductive coupling between a number of electrical coils (20, 21,21') and the subject's body in a way that the properties of said inductive coupling change if a pulse passes a screened volume underneath the sensing position, wherein the number of electrical coils comprises a field coil (20) producing a primary magnetic field and at least one receiving coil (21, 21') for screening the primary magnetic field, the axis of the at least one receiving coil (20) being perpendicular to the axis of the field coil (21, 21'); and
- detecting said property changes of the inductive coupling.

**7.** Method for determining the heart rate of a subject, the method comprising the steps of:

- detecting the passing of at least two successive pulse waves using the method as claimed in claim 6,
- measuring the time interval between said pulse waves, and determining the heart rate.

**8.** Method for determining the blood pressure of a subject, the method comprising the steps of:

- detecting the passing of a pulse wave at two spaced apart sensing locations using the method as claimed in claim 6,
- measuring the pulse transit time between said sensing locations, and determining the blood pressure.

**9.** A computer program for detecting the passing of a pulse wave from a subject's arterial system using the method disclosed in claim 6, during which an inductive coupling between a number of electrical coils (20, 21,21') and the subject's body in generated in a way that the properties of said inductive coupling change if a pulse passes a screened volume underneath the sensing position, wherein the number of electrical coils comprises a field coil (20) for producing a primary magnetic field and at least one receiving coil (21,21') for screening the primary magnetic field, the axis of the at least one receiving coil (21, 21') being perpendicular to the axis of the field coil (20); the program comprising computer instructions to detect said property changes of the inductive coupling, when the computer program is executed in a computer.


**Patentansprüche**

**1.** Sensor zur Erkennung einer vom arteriellen System (22) einer Person ausgehenden Impulswelle, wobei der Sensor dafür ausgelegt ist, an einer Erfassungsposition auf dem Äußeren des Körpers der Person angeordnet zu werden, wobei der Sensor Folgendes umfasst:

- eine Anzahl von elektrischen Spulen (20, 21, 21') zum Erzeugen einer induktiven Kopplung mit dem Körper der Person auf eine Weise, dass die Eigenschaften der genannten induktiven Kopplung sich verändern, wenn eine Impulswelle ein abgeschirmtes Volumen unterhalb der Erfassungsposition durchquert, und
- eine Schaltung (100, 101, 102, 103), die mit der Anzahl von elektrischen Spulen verbunden ist, wobei die genannte Schaltung dafür ausgelegt ist, die genannten Eigenschaftsänderungen der induktiven Kopplung zu erkennen,

**dadurch gekennzeichnet, dass** die Anzahl der elektrischen Spulen eine Feldspule (20) zum Erzeugen eines primären Magnetfelds und mindestens eine Empfangsspule (21, 21') zum Abschirmen des primären Magnetfelds umfasst, wobei die Achse der mindestens einen Empfangsspule senkrecht zu der Achse der Feldspule verläuft.

2. Sensor nach Anspruch 1, **dadurch gekennzeichnet, dass** die mindestens eine Empfangsspule (21, 21') eine einzelne elektrische Spule umfasst, deren elektrische Eigenschaften sich ändern, wenn eine Impulswelle die Erfassungsposition durchquert.

3. Sensor nach Anspruch 1, **dadurch gekennzeichnet, dass** die mindestens eine Empfangsspule (21, 21') zwei separate elektrische Spulen umfasst, deren elektrische Eigenschaften sich ändern, wenn eine Impulswelle die Erfassungsposition durchquert.

4. Nicht-invasives Messsystem, das dafür ausgelegt ist, an dem Äußeren des Körpers der Person angebracht zu werden, **dadurch gekennzeichnet, dass** es einen Sensor nach Anspruch 1 umfasst, wobei das System dafür ausgelegt ist, Informationen über die Herzfrequenz der Person zu liefern.

5. Nicht-invasives Messsystem, das dafür ausgelegt ist, an dem Äußeren des Körpers der Person angebracht zu werden und das zwei Sensoren nach Anspruch 1 umfasst, wobei die Erfassungspositionen der genannten Sensoren durch einen Abstand voneinander getrennt sind, wobei das System dafür ausgelegt ist, Informationen über den Blutdruck der Person zu liefern.

6. Verfahren zur Erkennung einer vom arteriellen System (22) einer Person ausgehenden Impulswelle, wobei das Verfahren die folgenden Schritte umfasst:

- Erzeugen einer induktiven Kopplung zwischen einer Anzahl von elektrischen Spulen (20, 21, 21') und dem Körper der Person auf eine Weise, dass die Eigenschaften der genannten induktiven Kopplung sich verändern, wenn ein Impuls ein abgeschirmtes Volumen unterhalb der Erfassungsposition durchquert, wobei die Anzahl der elektrischen Spulen eine Feldspule (20) zum Erzeugen eines primären Magnetfelds und mindestens eine Empfangsspule (21, 21') zum Abschirmen des primären Magnetfelds umfasst, wobei die Achse der mindestens einen Empfangsspule (21, 21') senkrecht zu der Achse der Feldspule (20) verläuft; und
- Erkennen der genannten Eigenschaftsänderungen der induktiven Kopplung.

7. Verfahren zum Ermitteln der Herzfrequenz einer Person, wobei das Verfahren die folgenden Schritte umfasst:

- Erkennen des Passierens von mindestens zwei aufeinanderfolgenden Impulswellen unter Verwendung des Verfahrens nach Anspruch 6,
- Messen des Zeitintervalls zwischen den genannten Impulswellen, und Ermitteln der Herzfrequenz.

8. Verfahren zum Ermitteln des Blutdrucks einer Person, wobei das Verfahren die folgenden Schritte umfasst:

- Erkennen des Passierens einer Impulswelle an zwei durch einen Abstand voneinander getrennten Erfassungspositionen unter Verwendung des Verfahrens nach Anspruch 6,
- Messen der Impulswellenlaufzeit zwischen den genannten Erfassungspositionen, und Ermitteln des Blutdrucks.

9. Computerprogramm zur Erkennung einer vom arteriellen System einer Person ausgehenden Impulswelle unter Verwendung des in Anspruch 6 beschriebenen Verfahrens, während dem eine induktive Kopplung zwischen einer Anzahl von elektrischen Spulen (20, 21, 21') und dem Körper der Person auf eine Weise erzeugt wird, dass die Eigenschaften der genannten induktiven Kopplung sich verändern, wenn ein Impuls ein abgeschirmtes Volumen unterhalb der Erfassungsposition durchquert, wobei die Anzahl der elektrischen Spulen eine Feldspule (20) zum Erzeugen eines primären Magnetfelds und mindestens eine Empfangsspule (21, 21') zum Abschirmen des primären Magnetfelds umfasst, wobei die Achse der mindestens einen Empfangsspule (21, 21') senkrecht zu der Achse der Feldspule (20) verläuft; wobei das Programm Computeranweisungen umfasst, um die genannten Eigenschaftsänderungen der induktiven Kopplung zu erkennen, wenn das Computerprogramm in einem Computer ausgeführt wird.

**Revendications**

1. Capteur pour détecter le passage d'une onde pulsative à partir du système artériel d'un sujet (22), le capteur étant adapté pour être situé à une position de détection sur l'extérieur du corps du sujet, dans lequel le capteur comprend :

   - un nombre de bobines électriques (20, 21, 21') pour générer un couplage inductif au corps du sujet de manière telle que les propriétés dudit couplage inductif changent si une onde pulsative passe par un volume examiné sous la position de détection, et
   - un circuit (100, 101, 102, 103) connecté au nombre de bobines électriques, ledit circuit étant adapté pour détecter lesdits changements de propriété du couplage inductif,
   **caractérisé en ce que** le nombre de bobines électriques comprend une bobine de champ (20) pour produire un champ magnétique primaire et au moins une bobine réceptrice (21, 21') pour examiner le champ magnétique primaire, l'axe de l'au moins une bobine réceptrice étant perpendiculaire à l'axe de la bobine de champ.

2. Capteur selon la revendication 1, **caractérisé en ce que** l'au moins une bobine réceptrice (21, 21') comprend une seule bobine électrique, dont les propriétés électriques sont changées si une onde pulsative passe par la position de détection.

3. Capteur selon la revendication 1, **caractérisé en ce que** l'au moins une bobine réceptrice (21, 21') comprend deux bobines électriques séparées, dont les propriétés électriques sont changées si une onde pulsative passe par la position de détection.

4. Système de mesure non effractif, adapté pour être attaché à l'extérieur du corps du sujet, **caractérisé en ce qu'**il comprend un capteur selon la revendication 1, le système étant adapté pour fournir des informations concernant la fréquence cardiaque du sujet.

5. Système de mesure non effractif, adapté pour être attaché à l'extérieur du corps du sujet, comprenant deux capteurs selon la revendication 1, les positions de détection desdits capteurs étant espacées l'une de l'autre, le système étant adapté pour fournir des informations concernant la pression sanguine du sujet.

6. Procédé pour détecter le passage d'une onde pulsative à partir du système artériel d'un sujet (22), le procédé comprenant les étapes de :

   - la génération d'un couplage inductif entre un nombre de bobines électriques (20, 21, 21') et le corps du sujet de manière telle que les propriétés dudit couplage inductif changent si un pouls passe par un volume examiné sous la position de détection, dans lequel le nombre de bobines électriques comprend une bobine de champ (20) produisant un champ magnétique primaire et au moins une bobine réceptrice (21, 21') pour examiner le champ magnétique primaire, l'axe de l'au moins une bobine réceptrice (21, 21') étant perpendiculaire à l'axe de la bobine de champ (20), et
   - la détection desdits changements de propriété du couplage inductif

7. Procédé pour déterminer la fréquence cardiaque d'un sujet, le procédé comprenant les étapes de :

   - la détection du passage d'au moins deux ondes pulsatives successives en utilisant le procédé selon la revendication 6,
   - la mesure de l'intervalle de temps entre lesdites ondes pulsatives, et la détermination de la fréquence cardiaque.

8. Procédé pour déterminer la pression sanguine d'un sujet, le procédé comprenant les étapes de :

   - la détection du passage d'une onde pulsative à deux emplacements de détection espacés l'un de l'autre en utilisant le procédé selon la revendication 6,
   - la mesure du temps de transit pulsatif entre lesdits emplacements de détection, et la détermination de la pression sanguine.

**9.** Programme d'ordinateur pour détecter le passage d'une onde pulsative à partir du système artériel d'un sujet en utilisant le procédé selon la revendication 6, durant lequel un couplage inductif entre un nombre de bobines électriques (20, 21, 21') et le corps du sujet dans généré de manière telle que les propriétés dudit couplage inductif changent si un pouls passe par un volume examiné sous la position de détection, dans lequel le nombre de bobines électriques comprend une bobine de champ (20) pour produire un champ magnétique primaire et au moins une bobine réceptrice (21, 21') pour examiner le champ magnétique primaire, l'axe de l'au moins une bobine réceptrice (21, 21') étant perpendiculaire à l'axe de la bobine de champ (20) ; le programme comprenant des instructions d'ordinateur pour détecter lesdits changements de propriété du couplage inductif, lorsque le programme d'ordinateur est exécuté dans un ordinateur.

**FIG. 1**

**FIG. 2**

FIG. 3

FIG. 4

FIG. 5

FIG. 6

FIG. 7

FIG. 8

$I_{max}(f)$

200

$U$

$I = I_{Inphase}$

$I_{Quadrature} = 0$

$\varphi = 0°$

f

f. (resonance)

FIG. 9

$I_{max}(f)$

200

$U$

$I$

$I_{Inphase}$

$\varphi$

$I_{Quadrature}$

f

$f_2\ (I_{Quadrature} = I_{Inphase},\ \varphi = 45°)$

FIG. 10

FIG. 11

FIG. 12

FIG. 13

FIG. 14

FIG. 15

**EP 2 049 012 B1**

## REFERENCES CITED IN THE DESCRIPTION

**Patent documents cited in the description**

- US 4425920 A
- US 6648828 B
- US 5309916 A
- EP 1350460 A